# FASCICULE DE BREVET EUROPEEN

(11) **EP 3 629 000 B1**
(45) Date de publication et mention de la délivrance du brevet: **04.08.2021**
(21) Numéro de dépôt: 19199433.4
(22) Date de dépôt: 25.09.2019
(51) Int. Cl.: C12M 1/00, G01N 1/40, B01L 3/00

(54) **PROCÉDÉ DE PRÉPARATION D'UN ÉCHANTILLON BIOLOGIQUE**
VERFAHREN ZUR PRÄPARATION EINER BIOLOGISCHEN PROBE
METHOD FOR PREPARING A BIOLOGICAL SAMPLE

(30) Priorité: 28.09.2018 FR 1858926
(43) Date de publication de la demande: 01.04.2020
(73) Titulaire: Commissariat à l'Énergie Atomique et aux Énergies Alternatives, 75015 Paris (FR); Service Departemental Metropolitain d'Incendie et de Secours (SDMIS), 69003 Lyon 3 (FR)
(72) Inventeur: BOURDAT, Anne-Gaëlle, 38054 Grenoble Cedex 9 (FR); WENISCH, Grégory, 69003 LYON 3 (FR)
(74) Mandataire: INNOV-GROUP

(56) Documents cités:
- US-A1- 2006 257 992
- US-A1- 2017 268 041
- US-B2- 7 785 535
- YADONG TANG ET AL: "Microfluidic device with integrated microfilter of conical-shaped holes for high efficiency and high purity capture of circulating tumor cells", SCIENTIFIC REPORTS, vol. 4, no. 1, 13 août 2014 (2014-08-13), XP055660349, DOI: 10.1038/srep06052

## Description

### Domaine technique de l'invention

La présente invention se rapporte à un procédé de préparation d'un échantillon biologique pour en extraire des espèces biologiques à analyser, mis en œuvre à l'aide d'un dispositif de préparation.

Le procédé se caractérise par le fait qu'il permet, dans le même dispositif et selon les étapes qui sont mises en œuvre, à partir d'un même échantillon biologique comprenant des espèces biologiques (bactéries, virus, moisissures, levures, cellules, toxines ou tissus animal ou végétal), de préparer plusieurs échantillons contenants chacun des espèces biologiques d'intérêt différentes (molécules d'ADN et protéines membranaires notamment).

L'invention concerne également un système de préparation utilisé pour mettre en œuvre ledit procédé.

### Etat de la technique

Le document US2004/142463A1 décrit une solution de préparation d'un échantillon, notamment un échantillon sanguin.

La demande de brevet EP3222989A1 décrit pour sa part un dispositif permettant de lyser des espèces biologiques présentes dans un échantillon, notamment pour extraire des molécules d'ADN en vue d'une détection par amplification de type PCR. En revanche, pour la préparation d'un échantillon en vue d'une détection immunologique, il existe peu de solution vraiment adaptée.

De telles solutions sont par exemple décrites dans les publications suivantes : US7785535 B2, YADONG TANG ET AL : "Microfluidic device with integrated microfilter of conical-shaped holes for high efficiency and high purity capture of circulating tumor cells", SCIENTIFIC REPORTS, vol. 4, no.1, 13 août 2014 (2014-08-13), DOI:10.1038/srep06052. Pourtant les dispositifs utilisés dans ces publications sont très complexes.

L'autre exemple d'appareil qui permet d'inverser le flux fluidique pour éluer et collecter des espèces biologiques à l'aide d'un filtre est décrit dans le document US 2066/257992 A1.

En règle générale, les tests de détection sont réalisés directement sur les échantillons, sans étape de concentration ni de purification. C'est le cas pour les échantillons comme l'urine, les "swab", échantillons de l'agroalimentaire, eau... Pour le sang, une étape de filtration ou centrifugation est réalisée pour extraire du sérum ou plasma. Or pour mettre en place une réaction de type immunologique qui soit efficace, il est essentiel de :
- Connaître la quantité d'espèces biologiques ciblées ;
- Maitriser la salinité de l'échantillon ;
- Maitriser le pH lors du processus ;

Par ailleurs, la préparation d'un échantillon pour une détection immunologique et la préparation d'un échantillon pour une extraction d'ADN sont souvent très différentes au niveau des protocoles mis en œuvre, même si certaines étapes peuvent être communes.

Il existe donc peu de procédé qui permette de réaliser simplement une préparation d'un échantillon en vue d'une détection de type immunologique.

Par ailleurs, il n'existe pas de procédé qui puisse permettre, à partir d'un seul dispositif et d'un même échantillon, de réaliser une préparation d'un premier échantillon en vue d'une détection de type immunologique et une préparation d'un deuxième échantillon en vue d'une réaction par amplification.

### Exposé de l'invention

Ce but est atteint par un procédé de préparation d'un échantillon biologique comprenant des espèces biologiques, mis en œuvre dans un système de préparation, ledit système de préparation comprenant un dispositif qui comporte :
- Un boîtier comportant au moins une ouverture,
- Un premier canal ménagé dans ledit boîtier,
- Un deuxième canal ménagé dans ledit boîtier,
- Une chambre dans laquelle débouche le premier canal et le deuxième canal,
- Un filtre séparant ladite chambre en deux espaces distincts de manière à définir un premier espace dans lequel débouche ledit premier canal d'injection et un deuxième espace dans lequel débouche ledit deuxième canal, ledit filtre ayant une porosité adaptée pour retenir ledit matériel biologique à analyser,

Ledit procédé comportant les étapes suivantes :
- Injection de l'échantillon biologique sous la forme d'un fluide par le premier canal pour effectuer une concentration des espèces biologiques dans le premier espace de la chambre dudit dispositif,
- Lyse desdites espèces biologiques dans ladite chambre pour broyer lesdites espèces biologiques et séparer les membranes du matériel intracellulaire,
- Elution du matériel intracellulaire en injectant un tampon d'amplification à travers le premier canal du dispositif, pour éluer ledit matériel par le deuxième canal,
- Injection d'un fluide tampon immunologique par le deuxième canal du dispositif de manière à éluer au moins en partie lesdites espèces biologiques avec ledit fluide tampon immunologique.

Selon une particularité, le procédé comporte une étape de lavage après la concentration, mise en œuvre en injectant un tampon de lavage par ledit premier canal du dispositif, pour purifier lesdites espèces biologiques.

L'invention concerne également un système de préparation d'un échantillon biologique qui comporte des espèces biologiques, ledit système de préparation comprenant un dispositif de préparation qui comporte :
- Un boîtier comportant au moins une ouverture,
- Un premier canal ménagé dans ledit boîtier,
- Un deuxième canal ménagé dans ledit boîtier,
- Une chambre dans laquelle débouche le premier canal et le deuxième canal,
- Un filtre séparant ladite chambre en deux espaces distincts de manière à définir un premier espace dans lequel débouche ledit premier canal d'injection et un deuxième espace dans lequel débouche ledit deuxième canal, ledit filtre ayant une porosité adaptée pour retenir ledit matériel biologique à analyser

Ledit système comprenant un circuit fluidique connecté sur ledit dispositif, ledit circuit fluidique comprenant plusieurs vannes commandées et une unité de traitement et de commande configurée pour commander chaque vanne en vue de mettre en œuvre le procédé tel que défini ci-dessus.

Selon une particularité, le circuit fluidique comporte une première vanne agencée pour connecter une première extrémité du premier canal du dispositif à un canal d'injection ou à un dispositif de détection immunologique.

Selon une autre particularité, le circuit fluidique comporte une deuxième vanne et trois réservoirs et la deuxième vanne est agencée pour connecter ledit canal d'injection à l'un desdits trois réservoirs.

Selon une autre particularité, le circuit fluidique comporte une troisième vanne agencée pour connecter une première extrémité du deuxième canal à un dispositif de détection par amplification ou à un troisième canal.

Selon une autre particularité, le circuit fluidique comporte une quatrième vanne agencée pour connecter le troisième canal à un réservoir destiné à contenir un tampon de réaction immunologique ou à une poubelle.

L'invention concerne également l'utilisation du système tel que défini ci-dessus, pour réaliser la préparation d'un échantillon biologique en vue d'une détection de type immunologique.

L'invention concerne enfin l'utilisation du système tel que défini ci-dessus pour réaliser la préparation d'un échantillon biologique en vue d'une détection par amplification.

### Brève description des figures

D'autres caractéristiques et avantages vont apparaître dans la description détaillée qui suit faite en regard des dessins annexés dans lesquels :
- La figure 1A représente un exemple d'un système de préparation employé pour mettre en œuvre le procédé de préparation de l'invention ;
- La figure 1B montre, en vue de dessus, le dispositif de préparation employé dans le système de la figure 1A ;
- Les figures 2A à 2F illustrent les différentes étapes du procédé de l'invention, mises en œuvre à l'aide du système de la figure 1A.

### Description détaillée d'au moins un mode de réalisation

Le procédé de l'invention se destine à la préparation d'un échantillon biologique.

L'échantillon biologique se présente par exemple sous la forme d'un fluide qui contient des espèces biologiques. Par espèces biologiques, on entend notamment des micro-organismes, des cellules, des spores, des toxines... A titre d'exemple, une espèce biologique peut comporter une membrane et un matériel intracellulaire tel que par exemple des molécules d'acide nucléique (ARN, ADN) issues d'une cellule, des protéines, des lipopolysaccharides (LPS), des acides lipotéichoïques (LTA)...

Par fluide, on entend notamment un liquide, un gaz... Le liquide pourra présenter différents degrés de viscosité et pourra par exemple se présenter sous la forme d'une pâte ou d'un gel.

Le procédé permet notamment de préparer un échantillon pour une détection immunologique et éventuellement également pour une réaction d'amplification, en utilisant un même dispositif de préparation et un même échantillon de départ.

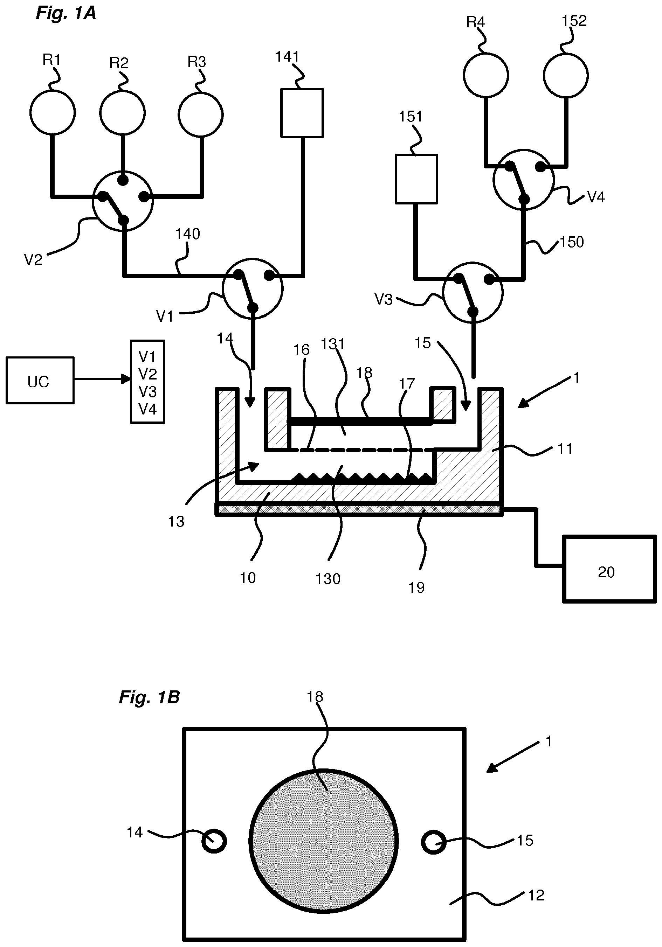

Le dispositif de préparation peut être intégré dans un système de préparation tel que celui représenté sur la figure 1A.

Le dispositif de préparation 1 comporte pour sa part un boîtier comprenant une paroi inférieure 10, une paroi latérale 11 et une paroi supérieure 12. Toutes les parois du boîtier seront préférentiellement réalisées dans un même matériau. Ce matériau sera notamment apte à pouvoir subir un chauffage dans une fourchette de température comprise entre 20°C et 100°C. Préférentiellement, certaines parois du boîtier, au moins sa paroi latérale 11, seront réalisées dans un matériau transparent. Préférentiellement, le matériau employé sera un plastique, par exemple de type PMMA (Poly(Méthacrylate de Méthyle)).

Le dispositif 1 comporte une chambre 13 ménagée dans le boîtier. Cette chambre représente l'emplacement dans lequel sont effectuées à la fois la purification/concentration, la lyse mécanique, la séparation et éventuellement la détection dans les espèces biologiques. La chambre 13 est fermée vers le bas par la paroi inférieure du boîtier.

Le dispositif comporte un premier canal 14 pour injecter des fluides dans la chambre ou pour évacuer des fluides en dehors de la chambre. Le premier canal 14 comporte une première extrémité comportant une ouverture ménagée par exemple à travers la paroi supérieure 12 du boîtier et une deuxième extrémité qui débouche dans ladite chambre 13. La première extrémité du premier canal 14 est par exemple agencée verticalement et sa deuxième extrémité débouche par exemple horizontalement dans la chambre 13. La première extrémité du premier canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type lueur pour y connecter une seringue ou adaptée pour y connecter un circuit fluidique tel que celui qui sera décrit ci-après.

Le dispositif comporte un deuxième canal 15. Ce deuxième canal 15 comporte également une première extrémité qui communique avec l'extérieur, formant une ouverture réalisée par exemple à travers la paroi supérieure du boîtier et une deuxième extrémité qui communique avec l'espace formé par la chambre 13. Par ce deuxième canal 15, on peut également injecter des fluides dans ladite chambre ou évacuer des fluides en dehors de ladite chambre. Sa première extrémité est par exemple agencée verticalement et sa deuxième extrémité horizontalement. La chambre 13 est placée entre le premier canal 14 et le deuxième canal 15. De manière identique, la première extrémité de ce deuxième canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type lueur pour y connecter une seringue ou adaptée pour y connecter un circuit fluidique tel que celui qui sera décrit ci-après.

Vers le haut, la chambre 13 peut être fermée par une membrane 18 souple et étirable, préférentiellement transparente. La paroi supérieure 12 du boîtier du dispositif comporte ainsi une ouverture qui est recouverte de manière hermétique par ladite membrane 18. Ladite membrane est ainsi ancrée dans le boîtier par toute solution de fixation adaptée, par exemple par collage. Cette membrane 18 sera par exemple composée d'un film, par exemple de type MicroAmp, 3M (marques déposées), d'épaisseur, de dimensions et de constitution adaptées pour se déformer de manière élastique, par rapport à ses points d'ancrage, notamment jusqu'au fond de la chambre 13.

Par le terme "transparent", on entend que le matériau employé est au moins partiellement transparent à la lumière visible, de manière à laisser passer au moins 80% de cette lumière. Il faut ainsi comprendre qu'il sera suffisamment transparent pour voir l'intérieur de la chambre 13, au moins le deuxième espace situé au-dessus du filtre.

Le dispositif comporte un filtre 16 agencé dans ladite chambre 13 et séparant ladite chambre 13 en deux espaces. Les deux espaces sont par exemple superposés et désignés ainsi espace inférieur 130 situé sous le filtre et espace supérieur 131 situé au-dessus du filtre. Ce filtre 16 est préférentiellement réalisé en tout ou partie sous la forme d'un film souple et fin, tiré dans l'espace formé par la chambre de manière à ne permettre le passage d'un espace à l'autre que par les pores du filtre 16. Le film présente une déformabilité élastique lui permettant de s'étirer lors de l'exercice d'une force d'appui dans une direction sensiblement verticale, cette déformabilité élastique ayant un niveau suffisant pour atteindre la surface inférieure de la chambre 13. Le filtre 16 présente un diamètre moyen de pores compris entre 0.2 µm et 50 µm, par exemple compris entre 0.2 µm et 1 µm pour la séparation de microorganismes. Le diamètre des pores est bien entendu adapté pour assurer une séparation entre différentes espèces biologiques présentes dans l'échantillon. On verra que, après une étape de lyse et de séparation par le filtre 16, le matériel intracellulaire (par exemple les molécules d'ADN) peut passer par élution au-dessus du filtre 16, dans l'espace supérieur 131 de la chambre, tandis que les membranes restent au-dessous du filtre, dans l'espace inférieur 130 de la chambre. Le filtre 16 sera par exemple composé d'un film d'épaisseur, de dimensions et de constitution adaptée pour se déformer jusqu'au fond de la chambre 13 par rapport à ses points d'ancrage. Selon un mode de réalisation particulier, le filtre pourra être aussi réalisé dans un matériau transparent, par exemple avec les mêmes caractéristiques de transparence que la membrane. Pour des bactéries, le filtre peut présenter un diamètre de pores allant de 0.2 à 2µm pour retenir les bactéries. Après lyse, l'ADN peut être élué à travers le filtre.

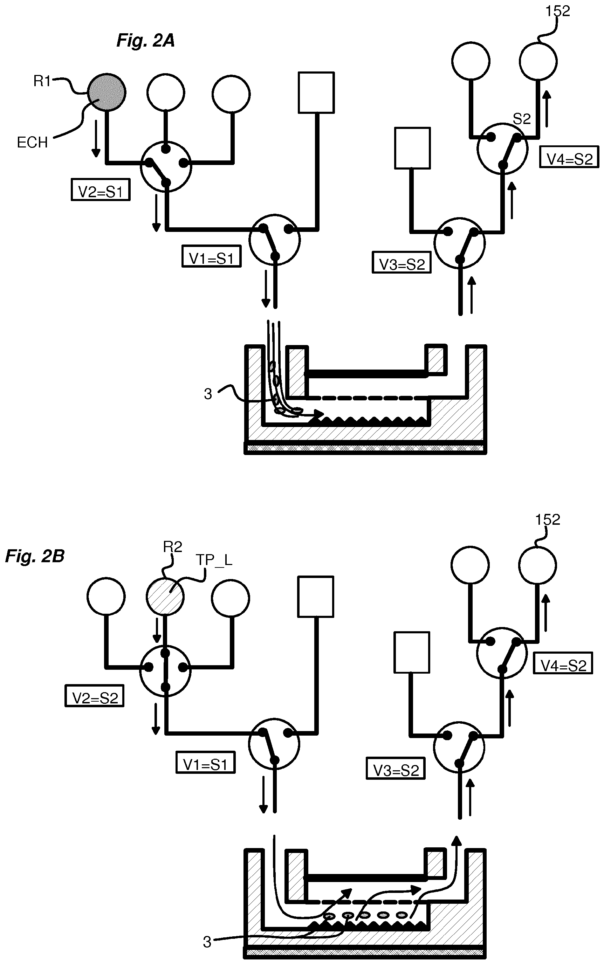

Le dispositif peut avantageusement comporter une surface d'appui rugueuse 17 agencée sur le fond de la chambre 13. Cette surface d'appui rugueuse 17 s'étend sur une partie majoritaire du fond de la chambre. Elle comporte un paramètre de rugosité de surface moyen compris entre 0.1 µm et 10 µm, préférentiellement compris entre 0.2 µm et 3 µm. Cette surface d'appui rugueuse 17 est destinée à permettre une lyse mécanique des espèces biologiques présentes dans un échantillon biologique placé dans le dispositif. Préférentiellement, la lyse mécanique est réalisée en broyant lesdites espèces biologiques, par abrasion sur ladite surface d'appui rugueuse. L'opération de broyage est mise en œuvre par un mouvement de friction des espèces biologiques contre la surface d'appui rugueuse, en employant un organe de broyage adapté. Cet organe sera par exemple une spatule 2 (voir figure 2B) ou une tige, par exemple en matériau plastique ou métallique. Cet organe est appliqué de l'extérieur de la chambre 13 et son extrémité est appliquée contre la surface externe de la membrane 18 de manière à étirer la membrane 18 et le filtre vers le fond de la chambre et ainsi frictionner les espèces biologiques présentes dans un échantillon contre la surface d'appui rugueuse.

Préférentiellement, le boîtier peut avantageusement intégrer des moyens de chauffage de l'espace interne de la chambre, composés par exemple d'au moins une résistance 19 chauffante, comme représenté sur les figures annexées. La résistance est par exemple fixée sous la paroi inférieure du boîtier. Une source d'alimentation 20 sera par exemple prévue pour alimenter la résistance 19. La source d'alimentation comportera par exemple une ou plusieurs piles électriques, fournissant suffisamment d'énergie pour chauffer la chambre à une température comprise dans la fourchette définie ci-dessus, c'est-à-dire de 20°C à 100°C. Bien entendu, d'autres moyens de chauffage pourraient être employés, comprenant par exemple une encre conductrice déposée par imprimerie ou sérigraphie sous la paroi inférieure du boîtier.

Ainsi, pour résumer, le dispositif peut avantageusement comporter la structure "multicouches" suivante :
- Une surface inférieure d'appui rugueuse 17,
- Un espace inférieur 130 de la chambre 13 situé au-dessus de la surface d'appui rugueuse 17,
- Un filtre 16 souple et étirable situé au-dessus de l'espace inférieur 130,
- Un espace supérieur 131 de la chambre 13 situé au-dessus du filtre 16,
- Une membrane 18 souple et étirable située au-dessus de l'espace supérieur 131, fermant hermétiquement la chambre et accessible depuis l'extérieur du dispositif.

Par ailleurs, le système de préparation, intégrant ledit dispositif, peut avantageusement comporter un circuit fluidique commandé, coopérant avec ledit dispositif pour mettre en œuvre le procédé.

Le circuit fluidique comporte avantageusement plusieurs vannes de commande permettant la mise en œuvre des différentes étapes du procédé de l'invention. Les vannes peuvent être de tous types, par exemple de type électrovanne ou autre solution technique.

Le circuit peut comporter une première vanne permettant de connecter la première extrémité du premier canal 14 à un canal d'injection 140 ou à un dispositif de détection immunologique 141 qui comporte une bandelette de détection immunologique.

La première vanne V1 peut prendre l'un des deux états distincts suivants :
- L'état S1 pour connecter uniquement le premier canal 14 au canal d'injection 140 ;
- L'état S2 pour connecter uniquement le premier canal 14 au dispositif de détection immunologique 141 ;
- L'état S3 fermé pour isoler la chambre 13 de l'extérieur ;

Le canal d'injection 140 peut être connecté à trois réservoirs R1, R2, R3 distincts, un premier réservoir R1 contenant l'échantillon biologique, un deuxième réservoir R2 contenant un tampon de lavage TP_L et un troisième réservoir R3 contenant un tampon d'amplification TP_AMP, tel qu'un réactif nécessaire à la mise en œuvre d'une réaction de type PCR ("Polymerase Chain Reaction"). Une deuxième vanne V2 à quatre voies peut être intercalée pour sélectionner le réservoir à connecter sur le canal d'injection 140.

La deuxième vanne V2 peut prendre l'un des trois états distincts suivants :
- L'état S1 pour connecter uniquement le réservoir R1 d'échantillon au canal d'injection 140 ;
- L'état S2 pour connecter uniquement le réservoir R2 de tampon de lavage TP_L au canal d'injection 140 ;
- L'état S3 pour connecter uniquement le réservoir R3 de tampon d'amplification TP_AMP au canal d'injection 140 ;

Le système peut comporter une troisième vanne V3 permettant de connecter la première extrémité du deuxième canal 15 à un dispositif de détection 151 par amplification ou à un troisième canal 150.

La troisième vanne V3 peut prendre l'un des deux états suivants :
- L'état S1 pour connecter le deuxième canal 15 uniquement au dispositif de détection 151 par amplification ;
- L'état S2 pour connecter le deuxième canal 15 au troisième canal 150 ;
- L'état S3 fermé pour isoler la chambre 13 de l'extérieur ;

Le système peut comporter une quatrième vanne V4 permettant de connecter ce troisième canal 150 à un réservoir R4 destiné à contenir un tampon de réaction immunologique TP_IM ou à une poubelle 152.

La quatrième vanne V4 peut prendre l'un des deux états suivants :
- L'état S1 pour connecter le troisième canal 150 au réservoir R4 de tampon de réaction immunologique TP_IM ;
- L'état S2 pour connecter le troisième canal à la poubelle 152 ;

Les différents réservoirs pourront être de tous types. Il pourra s'agir de seringue connectée directement sur le dispositif 1 par des embouts adaptés. Il pourra également s'agir de capsules fluidiques actionnables par pression (mécanique ou pneumatique). Les capsules peuvent être réalisées directement sur une carte de type micro-fluidique.

Le dispositif 1 ainsi que le circuit fluidique peuvent être réalisés sur une même carte micro-fluidique, par exemple en PMMA, comportant plusieurs couches pour réaliser les différents éléments du système.

Un tel système est donc adapté pour préparer un échantillon biologique pour mettre en œuvre plusieurs types de détection, notamment une détection immunologique ainsi qu'une réaction d'amplification (par exemple par PCR).

Le système peut comporter une unité de traitement et de commande UC destiné à exécuter une séquence de commande adaptée au procédé à mettre en œuvre. L'unité de traitement et de commande UC peut notamment envoyer des ordres de commande pour commuter chaque vanne du système d'un état à un autre et/ou envoyer des ordres de commande vers chaque réservoir pour commander une injection fluidique dans le système, en respectant les étapes du procédé.

Les figures 2A à 2E illustrent la mise en œuvre du procédé de l'invention et les différentes étapes de préparation de l'échantillon. Certaines étapes sont optionnelles et peuvent être omises selon l'objectif visé. L'unité de traitement et de commande UC peut envoyer les ordres de commande aux vannes du système. Sur ces figures, les flèches indiquent le sens de déplacement du fluide dans le système.

### Etape E1 - Figure 2A

Etats vannes :
V1=S1
V2=S1
V3=S2
V4=S2

L'échantillon ECH présent dans le réservoir R1 est injecté sous la forme d'un fluide par le premier canal 14 dans la chambre 13 du dispositif 1.

La partie de l'échantillon non retenue par le filtre 16 dans l'espace inférieur 130 de la chambre, traverse le filtre 16 et est évacué vers la poubelle 152 par le deuxième canal 15 du dispositif.

Les espèces biologiques 3 ciblées restent piégées dans l'espace inférieur 130 de la chambre 13.

### Etape E2 (optionnelle) - Figure 2B

Etats vannes :
V1=S1
V2=S2
V3=S2
V4=S2

Cette étape optionnelle permet de réaliser une purification des espèces biologiques 3 déjà présentes dans l'espace inférieur 130 de la chambre. Le tampon de lavage TP_L présent dans le réservoir R2 est injecté dans le premier canal 14 puis évacué du dispositif par le deuxième canal 15 vers la poubelle 152.

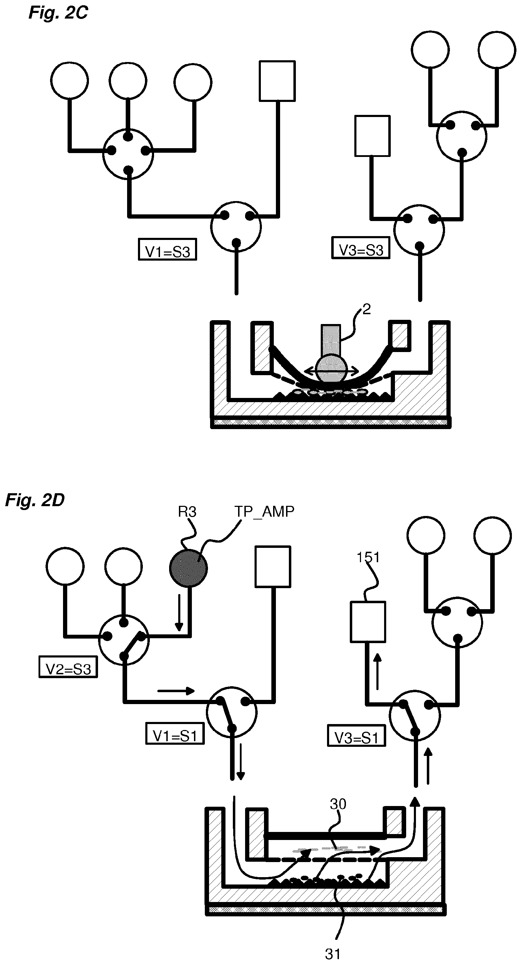

### Etape E3 (optionnelle) - Figure 2C

Etats vannes :
V1=S3
V3=S3

Dans cette étape, le premier canal 14 et le deuxième canal 15 du dispositif peuvent être totalement obturés en fermant les vannes V1 et V3 pour isoler la chambre 13 par rapport à l'extérieur.

Les espèces biologiques 3 de l'échantillon sont lysées par broyage contre la surface d'appui rugueuse 17 située au fond du dispositif 1.

La lyse permet de générer un lysat comprenant à la fois du matériel intracellulaire 30 et les membranes 31.

Cette étape est optionnelle.

### Etape E4 (optionnelle) - Figure 2D

Etats vannes :
V1=S1
V2=S3
V3=S1

Cette étape permet de réaliser une élution du matériel intracellulaire obtenu après lyse des espèces biologiques et présent dans l'espace inférieur 130 de la chambre 13 pour les amener vers un dispositif de détection par amplification. Un tampon d'amplification TP_AMP comprenant par exemple un réactif PCR, présent dans le réservoir R3, est injecté par le premier canal 14. Le matériel intracellulaire 30 est élué à travers le filtre alors que les membranes 31 restent piégées dans l'espace inférieur 130 de la chambre. Le matériel intracellulaire 30 est élué par le deuxième canal 15 vers un dispositif de détection 151 par amplification, par exemple pour subir une réaction d'amplification de type PCR.

Cette étape est optionnelle.

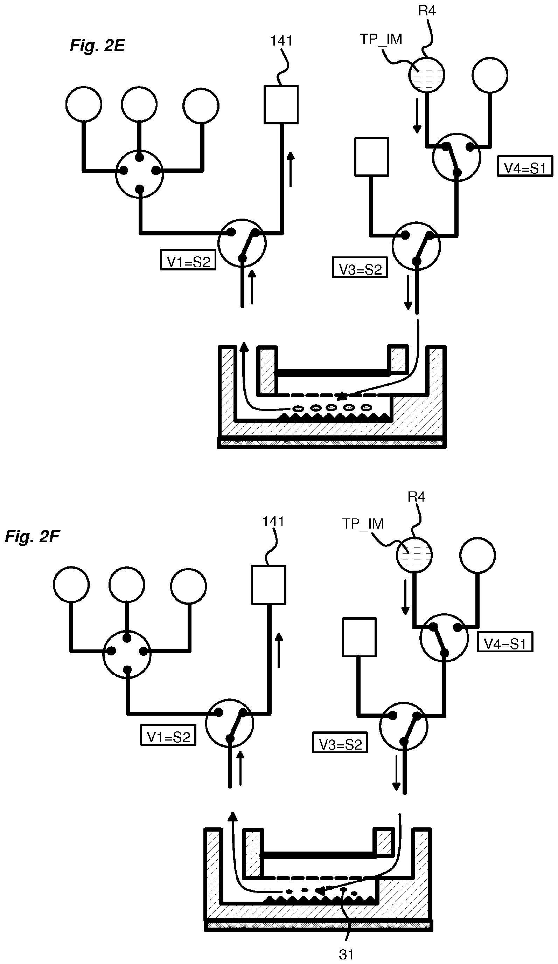

### Etape E5 - Figures 2E et 2F

Etats vannes :
V1=S2
V3=S2
V4=S1

Cette étape est mise en œuvre en injectant un fluide dans le sens inverse par rapport à précédemment. Un fluide tampon immunologique TP_IM présent dans le réservoir R4 est en effet injecté par le deuxième canal 15 permettant une élution des espèces biologiques vers le dispositif de détection immunologique 141.

Dans cette étape, selon que l'étape E3 évoquée ci-dessus est réalisée ou non, les espèces biologiques élués vers le dispositif de détection immunologique sont soit les membranes 31 des espèces biologiques (figure 2F - obtenues après la lyse de l'étape E3), soit les espèces biologiques 3 d'intérêt obtenues dès le départ juste après concentration réalisée lors de l'étape E1 (figure 2E).

De manière avantageuse, il sera également possible de prévoir une étape de séchage du filtre 16 après chaque filtrage ou passage d'un fluide à travers celui-ci. Cette étape de séchage peut être réalisée par passage d'un flux d'air à travers le filtre 16. Le flux d'air peut être créé entre les deux canaux 15, 16, par exemple par aspiration. Le flux d'air peut être créé dans un sens ou dans l'autre entre les deux canaux.

De manière non limitative, dispositif peut présenter les caractéristiques dimensionnelles suivantes :
- Un premier canal 14 composé d'un canal d'entrée de 1mm de diamètre x 3mm de hauteur, puis d'un canal de section rectangulaire de 1mmx15µm de 3mm de long ;
- Une chambre 13 composé d'un espace inférieur 130 pour concentration/lyse qui présente un diamètre de 8mmx150µm de hauteur et d'un espace supérieur pour élution d'un diamètre 8mmx300µm de hauteur ;
- Filtre 16 de porosité 0.6µm ou 0.8µm ou porosité adaptée à la cible à retenir (virus, levure, moisissure...) ;
- Un deuxième canal 15 composé d'un canal de section rectangulaire de 1mm x 150µm et de 3mm de long, puis d'un canal de 1mm de diamètre x3mm de hauteur ;

Le procédé de préparation l'invention, que ce soit pour une détection de type immunologique et/ou pour une détection par amplification, présente les avantages définis ci-dessous.
- Performances apportées par le procédé de préparation de l'invention, en amont d'une détection par amplification :
   ∘ Pas de perte de bactéries (pendant l'étape de concentration et de purification, des pertes inférieures à 1/10⁸ ont été constatées) ;
   ∘ Rendement de lyse amélioré (ex : sur B. anthracis : seulement 1 pour 10⁶ bactéries ont été non lysées) ;
   ∘ 100% de l'ADN théorique retrouvé en amplification ;
- Performances apportées par le procédé de préparation en amont d'une détection immunologique :
   ∘ Elimination des interférents de la détection immunologique (acide, base, sels...) ;
   ∘ Gain d'un logarithme si concentration d'un logarithme ;
   ∘ Possible lyse qui permet d'atteindre aussi les marqueurs membranaires intra cellulaires ;
   ∘ Diminution du risque pour l'opérateur en cas de tests sur bactéries pathogènes. Seulement 1 pour 10⁶ bactéries reste non lysées ;

Par ailleurs, on constate aussi les avantages suivants :
- Une préparation d'échantillon très simple ;
- Une préparation d'échantillon très rapide, pouvant être réalisée dans une durée inférieure à 10minutes ;
- Une solution qui ne divise pas l'échantillon : pas de fractionnement pour réaliser deux préparations indépendantes, permettant notamment de diminuer les risques pour l'opérateur ;
- Chaque échantillon préparé ressort directement dans son réactif d'analyse (pas de dilution, ni fractionnement de l'échantillon pour l'analyser). Toutes les cibles présentes dans l'échantillon sont engagées dans la réaction de détection (pas de dilution, d'utilisation d'une partie de l'éluât...) ;
- Une préparation d'échantillon qui permet à la fois de concentrer et de purifier ;
- Une lyse de très forte efficacité. Par exemple : Lyse de 100% des spores (espèces réputées difficiles à lyser). Lysat membranaire déposé sur bandelette ou ELISA ;
- Une sensibilité comparable ou même supérieure aux méthodes de référence ;

## Revendications

1. Procédé de préparation d'un échantillon biologique comprenant des espèces biologiques, mis en œuvre dans un système de préparation, ledit système de préparation comprenant un dispositif qui comporte :
- Un boîtier comportant au moins une ouverture,
- Un premier canal (14) ménagé dans ledit boîtier,
- Un deuxième canal (15) ménagé dans ledit boîtier,
- Une chambre (13) dans laquelle débouche le premier canal et le deuxième canal,
- Un filtre (16) séparant ladite chambre en deux espaces distincts de manière à définir un premier espace (130) dans lequel débouche ledit premier canal d'injection et un deuxième espace (131) dans lequel débouche ledit deuxième canal, ledit filtre (16) ayant une porosité adaptée pour retenir ledit matériel biologique à analyser,
Ledit procédé étant **caractérisé en ce qu'**il comporte les étapes suivantes :
- Injection de l'échantillon biologique sous la forme d'un fluide par le premier canal (14) pour effectuer une concentration des espèces biologiques dans le premier espace (130) de la chambre dudit dispositif,
- Lyse desdites espèces biologiques dans ladite chambre pour broyer lesdites espèces biologiques et séparer les membranes (31) du matériel intracellulaire (30),
- Elution du matériel intracellulaire (30) en injectant un tampon d'amplification (TP_AMP) à travers le premier canal (14) du dispositif, pour éluer ledit matériel par le deuxième canal (15),
- Injection d'un fluide tampon immunologique (TP_IM) par le deuxième canal (15) du dispositif de manière à éluer au moins en partie lesdites espèces biologiques avec ledit fluide tampon immunologique.

2. Procédé selon la revendication 1, **caractérisé en ce qu'**il comporte une étape de lavage après la concentration, mise en œuvre en injectant un tampon de lavage (TP_L) par ledit premier canal du dispositif, pour purifier lesdites espèces biologiques.

3. Système de préparation d'un échantillon biologique qui comporte des espèces biologiques, ledit système de préparation comprenant un dispositif de préparation qui comporte :
- Un boîtier comportant au moins une ouverture,
- Un premier canal (14) ménagé dans ledit boîtier,
- Un deuxième canal (15) ménagé dans ledit boîtier,
- Une chambre (13) dans laquelle débouche le premier canal et le deuxième canal,
- Un filtre (16) séparant ladite chambre en deux espaces distincts de manière à définir un premier espace dans lequel débouche ledit premier canal d'injection et un deuxième espace dans lequel débouche ledit deuxième canal, ledit filtre ayant une porosité adaptée pour retenir ledit matériel biologique à analyser,
Ledit système étant **caractérisé en ce qu'**il comporte un circuit fluidique connecté sur ledit dispositif, ledit circuit fluidique comprenant plusieurs vannes (V1-V4) commandées et une unité de traitement et de commande (UC) configurée pour commander chaque vanne en vue de mettre en œuvre le procédé tel que défini dans la revendication 1 ou 2.

4. Système selon la revendication 3, **caractérisé en ce que** le circuit fluidique comporte une première vanne (V1) agencée pour connecter une première extrémité du premier canal (14) du dispositif à un canal d'injection (140) ou à un dispositif de détection immunologique (141).

5. Système selon la revendication 4, **caractérisé en ce que** le circuit fluidique comporte une deuxième vanne (V2) et trois réservoirs (R1, R2, R3) et **en ce que** la deuxième vanne est agencée pour connecter ledit canal d'injection (140) à l'un desdits trois réservoirs.

6. Système selon l'une des revendications 3 à 5, **caractérisé en ce que** le circuit fluidique comporte une troisième vanne (V3) agencée pour connecter une première extrémité du deuxième canal (15) à un dispositif de détection (151) par amplification ou à un troisième canal (150).

7. Système selon la revendication 6, **caractérisé en ce que** le circuit fluidique comporte une quatrième vanne (V4) agencée pour connecter le troisième canal (150) à un réservoir (R4) destiné à contenir un tampon de réaction immunologique (TP_IM) ou à une poubelle (152).

8. Utilisation du système tel que défini dans l'une des revendications 3 à 7, pour réaliser la préparation d'un échantillon biologique en vue d'une détection de type immunologique.

9. Utilisation du système tel que défini dans l'une des revendications 3 à 7 pour réaliser la préparation d'un échantillon biologique en vue d'une détection par amplification.

## Patentansprüche

1. Verfahren zur Herstellung einer biologische Spezies umfassenden biologischen Probe, das in einem Herstellungssystem durchgeführt wird, wobei das Herstellungssystem eine Vorrichtung umfasst, die Folgendes umfasst:
- ein Gehäuse mit mindestens einer Öffnung,
- einen in dem Gehäuse hergestellten ersten Kanal (14),
- einen in dem Gehäuse hergestellten zweiten Kanal (15),
- eine Kammer (13), in die der erste Kanal und der zweite Kanal münden,
- ein Filter (16), das die Kammer in zwei getrennte Räume derart unterteilt, dass ein erster Raum (130), in den der erste Injektionskanal mündet, und ein zweiter Raum (131), in den der zweite Kanal mündet, gebildet werden, wobei das Filter (16) eine Porosität aufweist, die dafür ausgelegt ist, das zu analysierende biologische Material zurückzuhalten,
wobei das Verfahren durch die folgenden Schritte gekennzeichnet ist:
- Injektion der biologischen Probe in Form einer Flüssigkeit durch den ersten Kanal (14), um eine Konzentration der biologischen Spezies im ersten Raum (130) der Kammer der Vorrichtung durchzuführen,
- Lyse der biologischen Spezies in der Kammer, um die biologischen Spezies aufzubrechen und die Membranen (31) vom intrazellulären Material (30) zu trennen,
- Elution des intrazellulären Materials (30) durch Injizieren eines Amplifikationspuffers (TP_AMP) durch den ersten Kanal (14) der Vorrichtung, um das Material durch den zweiten Kanal (15) zu eluieren;
- Injektion einer immunologischen Pufferflüssigkeit (TP_IM) durch den zweiten Kanal (15) der Vorrichtung, um die biologischen Spezies mit der immunologischen Pufferflüssigkeit zumindest zum Teil zu eluieren.

2. Verfahren nach Anspruch 1, **dadurch gekennzeichnet, dass** es nach der Konzentration einen Waschschritt zum Reinigen der biologischen Spezies umfasst, der durch Injizieren eines Waschpuffers (TP_L) durch den ersten Kanal der Vorrichtung durchgeführt wird.

3. System zur Herstellung einer biologische Spezies umfassenden biologischen Probe, wobei das Herstellungssystem eine Herstellungsvorrichtung umfasst, umfassend:
- ein Gehäuse mit mindestens einer Öffnung,
- einen in dem Gehäuse hergestellten ersten Kanal (14),
- einen in dem Gehäuse hergestellten zweiten Kanal (15),
- eine Kammer (13), in die der erste Kanal und der zweite Kanal münden,
- ein Filter (16), das die Kammer in zwei getrennte Räume derart unterteilt, dass ein erster Raum, in den der erste Injektionskanal mündet, und ein zweiter Raum, in den der zweite Kanal mündet, gebildet wird, wobei das Filter eine Porosität aufweist, die dafür ausgelegt ist, das zu analysierende biologische Material zurückzuhalten,
- wobei das System **dadurch gekennzeichnet ist, dass** einen Flüssigkeitskreislauf umfasst, der mit der Vorrichtung verbunden ist, wobei der Flüssigkeitskreislauf mehrere gesteuerte Ventile (V1-V4) und eine Verarbeitungs- und Steuereinheit (UC) umfasst, die dafür ausgelegt ist, jedes Ventil so zu steuern, dass das Verfahren nach Anspruch 1 oder 2 durchgeführt wird.

4. System nach Anspruch 3, **dadurch gekennzeichnet, dass** der Flüssigkeitskreislauf ein erstes Ventil (V1) umfasst, das so angeordnet ist, dass es ein erstes Ende des ersten Kanals (14) der Vorrichtung mit einem Injektionskanal (140) oder einer immunologischen Nachweisvorrichtung (141) verbindet.

5. System nach Anspruch 4, **dadurch gekennzeichnet, dass** der Flüssigkeitskreislauf ein zweites Ventil (V2) und drei Reservoirs (R1, R2, R3) umfasst, und dadurch, dass das zweite Ventil so angeordnet ist, dass es den Injektionskanal (140) mit einem der drei Reservoirs verbindet.

6. System nach einem der Ansprüche 3 bis 5, **dadurch gekennzeichnet, dass** der Flüssigkeitskreislauf ein drittes Ventil (V3) umfasst, das so angeordnet ist, dass es ein erstes Ende des zweiten Kanals (15) mit einer Vorrichtung zum Nachweis durch Amplifikation (151) oder mit einem dritten Kanal (150) verbindet.

7. System nach Anspruch 6, **dadurch gekennzeichnet, dass** der Flüssigkeitskreislauf ein viertes Ventil (V4) umfasst, das so angeordnet ist, dass es den dritten Kanal (150) mit einem zum Enthalten eines Immunreaktionspuffer (TP_IM) bestimmten Reservoir (R4) oder mit einem Abfallbehälter (152) verbindet.

8. Verwendung des Systems nach einem der Ansprüche 3 bis 7 zum Durchführen der Herstellung einer biologischen Probe für einen immunologischen Nachweis.

9. Verwendung des Systems nach einem der Ansprüche 3 bis 7 zum Durchführen der Herstellung einer biologischen Probe für einen Nachweis durch Amplifikation.

## Claims

1. Process for preparing a biological sample comprising biological species, implemented in a preparation system, said preparation system comprising a device that includes:
- A housing with at least one opening,
- A first channel (14) provided in said housing,
- A second channel (15) provided in said housing,
- A chamber (13) into which the first channel and the second channel open,
- A filter (16) separating said chamber into two distinct spaces, so as to define a first space (130) into which said first injection channel opens and a second space (131) into which said second channel opens, said filter (16) having a porosity suitable for retaining said biological material to be analysed,
said process being **characterized in that** it comprises the following steps:
- Injection of the biological sample in the form of a fluid via the first channel (14) to concentrate biological species in the first space (130) of the chamber of said device,
- Lysis of said biological species in said chamber to grind said biological species and separate the membranes (31) from the intracellular material (30),
- Elution of the intracellular material (30) by injecting an amplification buffer (TP_AMP) through the first channel (14) of the device, to elute said material via the second channel (15),
- Injection of an immunological buffer fluid (TP_IM) via the second channel (15) of the device so as to at least partially elute said biological species with said immunological buffer fluid.

2. Process according to Claim 1, **characterized in that** it comprises a washing step after concentration, carried out by injecting a wash buffer (TP_L) via said first channel of the device, to purify said biological species.

3. Preparation system for a biological sample that comprises biological species, said preparation system comprising a preparation device that includes:
- A housing with at least one opening,
- A first channel (14) provided in said housing,
- A second channel (15) provided in said housing,
- A chamber (13) into which the first channel and the second channel open,
- A filter (16) separating said chamber into two distinct spaces, so as to define a first space into which said first injection channel opens and a second space into which said second channel opens, said filter having a porosity suitable for retaining said biological material to be analysed,
said system being **characterized in that** it comprises a fluid circuit connected to said device, said fluid circuit comprising several controlled valves (V1-V4) and a processing and control unit (UC) configured to control each valve in order to implement the process as defined in Claim 1 or 2.

4. System according to Claim 3, **characterized in that** the fluid circuit comprises a first valve (V1) arranged to connect a first end of the first channel (14) of the device to an injection channel (140) or to an immunological detection device (141).

5. System according to Claim 4, **characterized in that** the fluid circuit comprises a second valve (V2) and three tanks (R1, R2, R3) and **in that** the second valve is arranged to connect said injection channel (140) to one of said three tanks.

6. System according to one of Claims 3 to 5, **characterized in that** the fluid circuit comprises a third valve (V3) arranged to connect a first end of the second channel (15) to an amplification detection device (151) or to a third channel (150).

7. System according to Claim 6, **characterized in that** the fluid circuit comprises a fourth valve (V4) arranged to connect the third channel (150) to a tank (R4) intended to contain an immunoassay buffer (TP_IM) or to a waste bin (152).

8. Use of the system as defined in one of Claims 3 to 7, to prepare a biological sample for immunological detection.

9. Use of the system as defined in one of Claims 3 to 7, to prepare a biological sample for detection by amplification.
